# NEW EUROPEAN PATENT SPECIFICATION

(11) **EP 3 946 179 B2**
(45) Date of publication and mention of the opposition decision: **03.09.2025**
(45) Mention of the grant of the patent: 27.07.2022
(21) Application number: 20716885.7
(22) Date of filing: 25.03.2020
(51) Int. Cl.: A61F 5/445, A61L 28/00

(54) **OSTOMY POUCHES**
STOMABEUTEL
POCHES DE STOMIE

(30) Priority: 29.03.2019 GB 201904455
(43) Date of publication of application: 09.02.2022
(73) Proprietor: Salts Healthcare Limited, West Midlands B7 4AA (GB)
(72) Inventor: POWNER, Iain, Birmingham West Midlands B7 4AA (GB); TRETHEWAY, Lee, Birmingham West Midlands B7 4AA (GB); BARRON, Andrew, Swansea West Glamorgan SA2 8PP (GB); HILL, Donald, Swansea West Glamorgan SA2 8PP (GB); ALEXANDER, Shirin, Swansea West Glamorgan SA2 8PP (GB)
(74) Representative: Forresters IP LLP
(86) International application number: PCT/GB2020/050790
(87) International publication number: WO 2020/201718

(56) References cited:
- WO-A2-2018/041320
- GB-A- 2 273 052
- GB-A- 2 510 563
- US-A1- 2007 003 463
- US-A1- 2013 081 555
- AEROXIDE® ALU information sheet from Evonik Industries August2013
- HAAS ET AL: "Hybrid Inorganic-Organic Polymers Based on Organically Modified Si-Alkoxides", vol. 2, no. 9, 1 January 2000 (2000-01-01), pages 571 - 582, XP009124212, ISSN: 1438-1656, Retrieved from the Internet <URL:http://www3.interscience.wiley.com/cgi-bin/issn?DESCRIPTOR=PRINTISSN&VALUE=1438-1656>

## Description

### FIELD

This specification relates to ostomy pouches and, in particular, to ostomy pouches where at least a portion of an inner surface thereof is coated with hydrophobic particles to result in the inner surface having a water contact angle (WCA) of approximately 140° or greater. The hydrophobic particles disclosed herein are non-toxic and adhere well to polymeric films. Accordingly, the hydrophobic particles disclosed herein are particularly well suited for use in ostomy.

### BACKGROUND

Ostomy pouches are medical devices that provide a means for the collection of body waste from a stoma. Ostomy pouches are most commonly associated with colostomies, ileostomies and urostomies. Ostomy pouches typically comprise a collection bag and a baseplate (also known as a flange or a wafer). There are one-piece ostomy pouches where the collection bag and baseplate are supplied as a single item and there are two-piece ostomy pouches where the collection bag and baseplate are supplied as separate items for attaching to one another.

Ostomy pouches can be further divided into two basic types: open-end pouches and closed-end pouches. Open-end pouches typically comprise a drainable opening for the removal of body waste, e.g. into a toilet. The drainable opening may include a valve or the drainable opening may be opened and closed using a clip or a hook and loop fastener. Closed-end pouches are typically removed from the patient when full. The closed-end pouch may then be disposed of or emptied and cleaned ready for re-use. Ostomy pouches, particularly closed-end pouches, can suffer from the phenomenon of pancaking. This is where a vacuum occurs within the pouch and the internal surfaces of the side walls stick together. Consequently, the body waste is prevented from falling to the bottom of the bag. The trapped body waste can contaminate adhesive on the baseplate which can result in the ostomy pouch becoming detached from the patient.

Open-end pouches can be problematic when the drainable opening gets trapped with body waste. In such circumstances the open-end pouch can be challenging to drain and may need to be removed to clean thoroughly. In certain circumstances, however, it may not be possible to sufficiently clean the drainable opening which can mean that the open-end pouch needs to be replaced.

It is known to use hydrophobic materials to create surfaces that are difficult to wet, non-stick, self-cleanable and / or resistant to contamination. Such hydrophobic materials typically include waxes, fluorinated polymers, e.g. polytetrafluoroethylene (PTFE), organosilanes, etc.

However, known hydrophobic materials are not always suitable for use in ostomy because often they do not adhere well to polymeric films and / or they can be toxic, e.g. fluorine-containing.

For example document GB 2 510 563 proposes to solve the problem of pancaking by using PTFE to coat the internal of the ostomy pouch. Further, WO 2018/041320 shows an ostomy pouch wherein the walls of the pouch may be coated with different polymers, metal oxides or composite materials comprising nano-particles. In particular, Ormocer^{®} may contain oxides of metals (Zr, Ti, Al) bonded to hydrocarbon chains.

There is a commercial need for improved ostomy pouches that seek to overcome the above-described problems.

### SUMMARY OF INVENTION

Embodiments of the present invention seek to provide ostomy pouches that are simple and quick to clean, address the issues of pancaking and drainage, whilst at the same time are durable and non-toxic.

According to a first aspect of the invention, there is provided an ostomy pouch having a pair of opposed side walls, one of the side walls defining a stoma-receiving opening for, in use, receiving a part of a stoma, one or both of the side walls being formed of a polymeric film at least partially coated on an internal surface thereof with hydrophobic particles, the hydrophobic particles comprising:
a metal oxide core; and
a hydrocarbon chain having from 6 to 32 carbon atoms,
wherein the hydrocarbon chain is chemically bound to the metal oxide core, characterised in that the hydrocarbon chain is branched.

The side wall defining the stoma-receiving opening may be formed of the polymeric film and may comprise a first region which at least partially surrounds or surrounds the stoma-receiving opening, wherein the first region is coated with the hydrophobic particles.

The first region may have a peripheral edge located approximately 20 mm to approximately 80 mm (such as from approximately 20 mm to approximately 60 mm, e.g. 40 mm) from the centre of the stoma-receiving opening.

The side wall opposing the side wall defining the stoma-receiving opening may be formed of the polymeric film and may comprise a second region which substantially faces the stoma-receiving opening, wherein the second region is coated with the hydrophobic particles.

The diameter of the second region may be greater than the diameter of the stoma-receiving opening.

The diameter of the second region may be greater than, less than or approximately equal to the diameter of the first region.

The ostomy pouch may comprise a drainable opening at a lower end thereof, both side walls being formed of the polymeric film and comprising a third region which surrounds the drainable opening, wherein the third region is coated with the hydrophobic particles.

The drainable opening may include a valve for controlling the flow of body waste therefrom.

The internal surface of the valve may be coated with the hydrophobic particles.

Both side walls may be formed of the polymeric film and the entire internal surfaces thereof may be coated with the hydrophobic particles.

The average diameter of the hydrophobic particles may be less than or equal to approximately 200 nm.

The average diameter of the hydrophobic particles may be less than or equal to approximately 50 nm, e.g. less than or equal to approximately 20 nm.

The average diameter of the hydrophobic particles may be from approximately 8 nm to approximately 20 nm, such as from approximately 8 nm to approximately 15 nm.

The metal oxide core may comprise one or a combination of aluminium oxide, iron oxide, zinc oxide, and silicon oxide.

The hydrocarbon chain may be aliphatic.

The hydrocarbon chain is branched.

The hydrocarbon chain has from 6 to 32 carbons, such as from 6 to 24 carbons.

The hydrocarbon chain may be covalently bound to the metal oxide core via a functional group, e.g. an anionic functional group.

The functional group may comprise any one or a combination of hydroxide, carboxylate, phosphonate, phosphinate, thiolate and thiocarboxylate.

The hydrophobic particles may be free from fluorine.

The polymeric film may comprise a thermoplastic film. For instance, the polymeric film may comprise any one or more of a polyolefin, vinyl polymer, polyester and polyacetal film.

The polymeric film may comprise a co-extruded bilayer or multilayer film. For instance, the co-extruded bilayer or multilayer film may comprise layers of any one or a combination of polyethylene (PE), polypropylene (PP), acetal, acrylic, polyamide, polyvinyl chloride (PVC), ethylene vinyl acetate (EVA), polyvinylidene chloride (PVDC), polystyrene (PS), acrylonitrile butadiene styrene (ABS) and polycarbonate (PC).

The hydrophobic particles may be deposited (e.g. sprayed) onto the polymeric film. For instance, the hydrophobic particles may be mixed with a carrier, such as a volatile solvent, and the resultant mixture may be sprayed onto the polymeric film. The mixture of the hydrophobic particles and the carrier may form a solution or suspension. The mixture may have a hydrophobic particle concentration of from approximately 0.5 wt% to approximately 20 wt%, such as from approximately 0.5 wt% to approximately 10 wt%, such as from approximately 0.5 wt% to approximately 5 wt%, e.g. 0.5 wt% or 1 wt% or 2 wt% or 3 wt% or 4 wt% or 5 wt%.

The hydrophobic particles may be at least partially embedded in the polymeric film.

The polymeric film may be heated to enable the hydrophobic particles to become at least partially embedded therein to form a hydrophobic three-dimensional surface. Embedding of the hydrophobic particles may be enhanced by physical means, such as by rolling the heated polymeric film between rollers. During processing, the volatile solvent (where used) naturally evaporates and as the polymeric film cools the hydrophobic particles remain bound to the polymeric film such that they are not removed by washing.

The temperature to which the polymeric film is heated will vary depending on the type of polymeric film being utilised. In general, the polymeric film is heated to a temperature at which it starts to deform plastically. It is to be appreciated that the skilled person will know this temperature or will be able to determine this temperature through basic experimentation. For the EVA/EVA/PVDC/EVA/EVA five layer co-extruded thermoplastic film utilised in the below examples, the plastics deformation temperature was between approximately 80 °C and approximately 90 °C.

The hydrophobic particles and the polymeric film may be secured to one another by an adhesive, such as an epoxy resin. For instance, an adhesive may be deposited onto the polymeric film followed by the hydrophobic particles. Alternatively, the hydrophobic particles may be deposited onto the polymeric film followed by the adhesive. The hydrophobic particles may become at least partially embedded in the polymeric film, which itself provides the bond between the polymeric film and the hydrophobic particles. When the adhesive is cured, the result is a polymeric film having a hydrophobic three-dimensional surface.

The hydrophobic particles and the adhesive may be mixed and the resultant mixture may be deposited onto the polymeric film.

Where the hydrophobic particles and the adhesive are mixed, the mass ratio of the hydrophobic particles and the adhesive may be from approximately 1.0:1.0 to approximately 2.0:1.0. Mass ratios of hydrophobic particles and adhesive in this range have been found to produce polymeric films having particularly good hydrophobic surfaces, particularly where the metal oxide core comprises aluminium oxide.

The hydrophobic particles or the hydrophobic particle / adhesive mixture may be sprayed onto the polymeric film using a carrier, e.g. a volatile solvent, as described previously.

In all methods of attaching the hydrophobic particles to the polymeric film it has been found that the WCAs are not adversely affected even after the polymeric film has been immersed in or exposed to a solvent. Such treatment may result in some of the hydrophobic particles becoming removed from the polymeric film but the removal has a negligible effect on the WCA. The methods of attaching the particles and the methods of manufacturing an ostomy bag are not a part of the invention.

The hydrophobicity of the polymeric film may be tuned at different regions across the surface thereof. By this it is meant that a first region of the polymeric film may have an associated first WCA measurement and a second (or further) region of the polymeric film may have an associated second WCA measurement which differs from the first WCA measurement.

Tuning the hydrophobicity of the polymeric film may be achieved in a number of ways. For instance, more layers of hydrophobic particles may be deposited at the first region than at the second region. Accordingly, the first region will typically have a higher WCA measurement than the second region. Additionally or alternatively, a more concentrated mixture of hydrophobic particles may be deposited at the first region than at the second region. Accordingly, the first region will typically have a higher WCA measurement than the second (or further) region. The concentration of hydrophobic particles in the mixture may be adjusted by dilution with the solvent and / or a different species, e.g. a hydrophilic particle and / or an unfunctionalised metal oxide. Additionally or alternatively, different types of hydrophobic particles may be deposited at the respective first and second regions. Accordingly the first and second regions will typically have differing WCA measurements.

The present invention provides ostomy pouches formed from self-cleaning polymeric films. At least a portion of the inside surface of the ostomy pouch has a hydrophobic surface formed by securing hydrophobic particles to a polymeric film.

The methods of attaching the hydrophobic particles to the polymeric film result in a polymeric film having a three-dimensional surface structure which can achieve a WCA of approximately 140° or greater. Accordingly, ostomy pouches of the invention can offer improved self-cleaning properties, be resistant to contamination and / or be easier to clean. Ostomy pouches of the present invention are also considered to be more durable in terms of these properties and, hence longer living, when compared to known ostomy pouches due to improved adhesion between the hydrophobic particles and the polymeric film.

### BRIEF DESCRIPTION OF THE FIGURES

Embodiments will now be described by way of example only with reference to the accompanying drawings, in which:
Figure 1 is a representation of a hydrophobic particle;
Figure 2 shows a polymeric film having hydrophobic particles at least partially embedded therein;
Figure 3 shows a polymeric film comprising hydrophobic particles bonded thereto by an adhesive;
Figure 4 is a cross sectional view of an ostomy pouch according to a first embodiment of the invention;
Figure 5 is a cross sectional view of an ostomy pouch according to a second embodiment of the invention; and
Figure 6 is a cross sectional view of an ostomy pouch according to a third embodiment of the invention.

### DESCRIPTION OF EMBODIMENTS

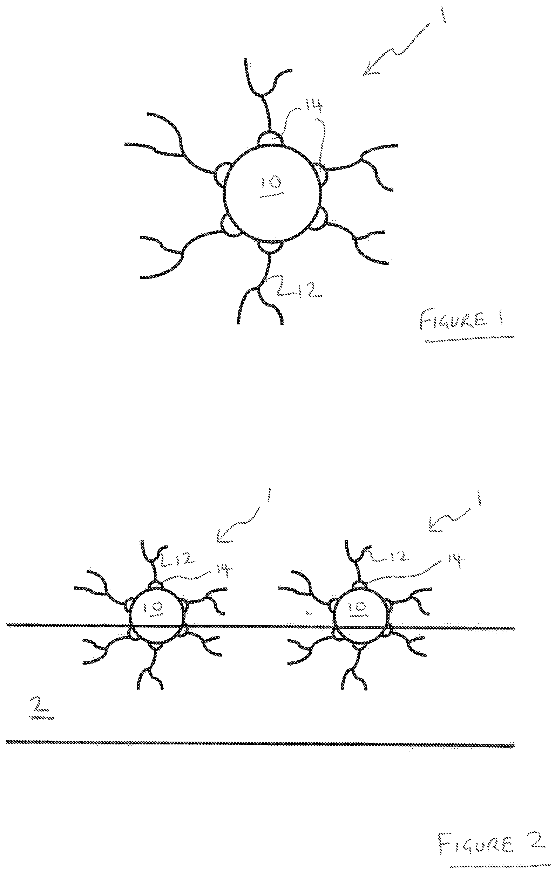

Referring to figure 1 there is shown a hydrophobic particle, represented generally at 1. The hydrophobic particle 1 comprises a metal oxide core 10 and six hydrocarbon chains 12. It is to be appreciated that in other embodiments the hydrophobic particle 1 may have greater than or less than six hydrocarbon chains 12. Each hydrocarbon chain 12 has from 6 to 32 carbon atoms. The hydrocarbon chains 12 are branched.

The hydrocarbon chains 12 are chemically bound to the metal oxide core 10. In some embodiments, the hydrocarbon chains 12 may be covalently bound to the metal oxide core 10 via a functional group 14. Suitable metal oxide cores 10 include aluminium oxide, iron oxide, zinc oxide, and silicon oxide.

The term "oxide" as used herein is intended to include oxide-hydroxides, hydroxides and also oxides having multiple metal oxidation states. For example, iron oxide may include Fe₃O₄ or Fe₂O₃ or a combination thereof.

In some embodiments, the hydrophobic particle 1 comprises a metal oxide core 10 having a hydrocarbon chain 12 covalently bound thereto by a carboxylate functional group 14. In other embodiments, alternative functional groups may be employed as long as a stable covalent interaction is formed between the metal oxide core 10 and the hydrocarbon chain 12. Alternative functional groups 14 may comprise any one or a combination of hydroxide, phosphonate, phosphinate, thiolate and thiocarboxylate.

In some embodiments, the hydrocarbon chain 12 may be aliphatic. In particular, the hydrocarbon chain 12 may be chosen from any suitable alkyl organic group as defined by the formula CₓH_{y}, where x and y are whole numbers and x is from 6 to 32. The hydrocarbon chain 12 has from 6 to 32 carbons, such as from 6 to 24 carbons.

The hydrocarbon chain 12 is branched. For instance, the hydrophobic particle 1 may be created by reaction of any one of isostearic acid (CH₃(CH₂)₁₆COOH) and 2-hexyldecanoic acid (CH₃(CH₂)₇CH[(CH₂)₅CH₃]CO₂H) with the metal oxide core 10.

Creation of the hydrocarbon chains 12 as described herein can provide the advantage that the resultant hydrophobic particles 1 are free from fluorine. This means that the hydrophobic particles 1 of the invention have environmental benefits in that they are less toxic when compared to prior art materials.

Referring now to figure 2, there is shown a polymeric film 2 having the hydrophobic particles 1 at least partially embedded therein.

In some embodiments, the polymeric film 2 is prepared by depositing, e.g. spraying, the hydrophobic particles 1 onto a heated surface thereof. The hydrophobic particles 1 may be dissolved or suspended in a solvent. The exposed surface of the polymeric film 2 may be heated until it is softened, followed by deposition of the solution or suspension onto the softened surface. After allowing the solvent to evaporate and the surface to cool the hydrophobic particles 1 become at least partially embedded in the polymeric film 2. The result is a polymeric film 2 having a stable, textured and hydrophobic surface. In some embodiments, the polymeric film 2 may be heated by radiation (for example, by infra-red lamps) or by conduction (for example, by placing the polymeric film 2 on a hot plate or by exposing to heated air). It is to be understood that any method that provides sufficient heating to soften the polymeric film 2 without compromising its integrity may be employed.

The choice of solvent is limited only by the need for the solvent to evaporate from the surface of the polymeric film 2. Suitable solvents include, but are not limited to, isopropanol, toluene and ethanol.

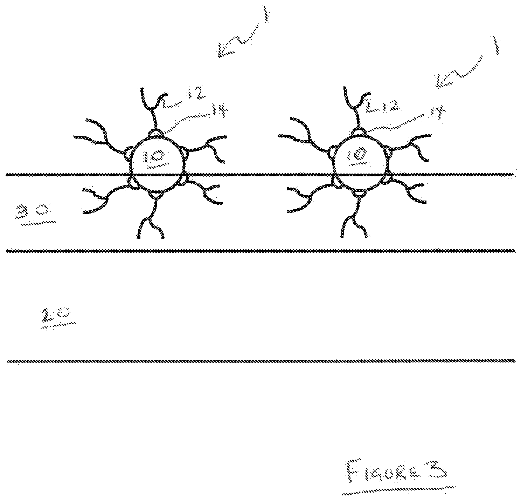

Referring now to figure 3, there is shown a polymeric film 20 where the hydrophobic particles 1 are secured thereto by an adhesive 30.

In some embodiments, the adhesive 30 may be an epoxy resin. The adhesive 30 may be applied to the polymeric film 20, followed by deposition of the solution or suspension containing the hydrophobic particles 1, or vice versa. The adhesive 30 is allowed to cure, at which point the hydrophobic particles 1 become bonded to the polymeric film 20 by virtue of being at least partially embedded in the adhesive 30.

In some embodiments, the hydrophobic particles 1 and the adhesive 30 may be mixed and the resultant mixture may be deposited, e.g. sprayed, onto the polymeric film 20.

Spraying may be effected by dissolving or suspending the mixture in a solvent and utilising a propellant or a compressor as is well known in the industry.

### Materials

The polymeric film may comprise a thermoplastic film, e.g. a polyethylene copolymer. The polymeric film used for all of the subsequent experiments was an EVA/EVA/PVDC/EVA/EVA five layer co-extrusion having a thickness of 75 micron.

Aluminium oxide (Al₂O₃) particles having an average diameter of 13 nm were purchased from Sigma-Aldrich.

Iron oxide (Fe₃O₄) particles having an average diameter of from 15 - 20 nm were purchased from Sigma-Aldrich.

Isostearic acid was purchased from Nissan Chemical Industries and was used without further purification.

Toluene and isopropanol were supplied by VWR Chemicals.

SP106 Multi-Purpose Epoxy Resin System 1 kg Slow Hardener was purchased from MB Fibreglass.

Spraycraft Universal Airbrush Propellant was used for the spray coating and was purchased from Axminster Tools and Machinery.

### Water contact angle (WCA) measurements

WCA measurements were used to study the wettability of the polymeric films. WCA measurements were obtained by depositing 4 µL droplets of H₂O onto the polymeric films. The values of the WCAs that are reported herein are the average of three measurements, recorded at different positions on the surfaces. Standard deviations are used to represent the uncertainties that are associated with these values.

### Comparative example 1

The WCA of the uncoated polymeric film (i.e. the clean EVA/EVA/PVDC/EVA/EVA five layer co-extruded film) was 88.3° ±1.7°.

### Comparative example 2

The polymeric film was coated with unfunctionalised Al₂O₃ particles. Deposition of the unfunctionalised Al₂O₃ particles onto the polymeric film was achieved through spray coating from 2% wt isopropanolic suspensions at ambient temperature. Three sprays were used to try to achieve maximum coverage of the polymeric film by the unfunctionalised Al₂O₃ particles.

Coating the polymeric film with the unfunctionalised Al₂O₃ particles at ambient temperature resulted in the surface thereof becoming superhydrophilic. Accordingly, it was not possible to accurately measure the WCA of the resultant polymeric film.

### Comparative example 3

The polymeric film was coated with unfunctionalised Fe₃O₄ particles. Deposition of the unfunctionalised Fe₃O₄ particles onto the polymeric film was achieved through spray coating from 2% wt isopropanolic suspensions at ambient temperature. Three sprays were used to try to achieve maximum coverage of the polymeric film by the unfunctionalised Fe₃O₄ particles.

The WCA of the resultant polymeric film was 107.2° ±3.4°.

### Example 1

Functionalised aluminium oxide (Al₂O₃) particles were synthesised as follows. Aluminium oxide (Al₂O₃) particles (d = 13 nm, 10.0 g, 98.0 mmol, 1.0 equiv.) were refluxed with isostearic acid (39.1 g, 137.3 mmol, 1.4 equiv.) in toluene (250 mL) for 24 hours. Once the reaction time had elapsed, the reaction mixture was collected and centrifuged at 5000 rpm for one hour. The solid was then recovered and centrifuged at 5000 rpm in isopropanol for one hour. Following this, the solid was centrifuged in ethanol at 5000 rpm for one hour three further times, and then dried at 80 °C for six hours.

The polymeric film was coated with the functionalised Al₂O₃ particles. Deposition of the functionalised Al₂O₃ particles onto the polymeric film was achieved through spray coating from 2% wt isopropanolic suspensions at ambient temperature. Three sprays were used to try to achieve maximum coverage of the polymeric film by the functionalised Al₂O₃ particles.

The WCA of the resultant polymeric film was 151.1° ±1.0°.

### Example 2

Functionalised iron oxide (Fe₃O₄) particles were synthesised as follows. Iron oxide (Fe₃O₄) particles (d = 15 - 20 nm, 5.0 g, 21.6 mmol, 1.0 equiv.) were refluxed in toluene (100 mL) with isostearic acid (18.4 g, 64.7 mmol, 3.0 equiv.) for approximately twenty-four hours, under mechanical stirring. Once the reaction time had elapsed, the mixture was centrifuged at 5000 rpm for one hour. Following this, the solid was recovered and dried at 80 °C for six hours.

The polymeric film was coated with the functionalised Fe₃O₄ particles. Deposition of the functionalised Fe₃O₄ particles onto the polymeric film was achieved through spray coating from 2% wt isopropanolic suspensions at ambient temperature. Three sprays were used to try to achieve maximum coverage of the polymeric film by the functionalised Fe₃O₄ particles.

The WCA of the resultant polymeric film was 151.9° ±2.1°.

### Example 3

The polymeric film was heated and coated with the functionalised Al₂O₃ particles described in Example 1. The functionalised Al₂O₃ particles were spray coated onto the polymeric film once it had been softened as a result of heating. Heating of the polymeric film was accomplished as follows. First, the polymeric film was physically attached at its edges to the surface of a glass petri dish. The purpose of this was to secure the polymeric film in order to limit the extent to which it changed shape during the heating process. Heat was then applied to the petri dish until physical deformation of the polymeric film was observed. Once physical deformation of the polymeric film was observed, deposition of the functionalised Al₂O₃ particles onto the polymeric film was achieved through spray coating. The functionalised Al₂O₃ particles were spray coated from a 2.0 %wt suspension. Five sprays were used to try to achieve maximum coverage of the polymeric film by the functionalised Al₂O₃ particles. Following each spray, the polymeric film was continually heated in order to accelerate the removal of the isopropanol. Further spray coating was performed on the polymeric film when no liquid was observed its surface. The temperature of the polymeric film was not measured prior to spray coating. However, it was observed that the polymeric film would start to deform plastically when heated to between 80-90 °C.

The WCA of the resultant polymeric film was 142.0° ±3.9°.

Although this value is slightly lower than when the functionalised Al₂O₃ particles were deposited onto the polymeric film at ambient temperature (Example 1), it is noteworthy that water droplets would readily roll off the coated polymeric film. Accordingly, this suggests that heating of the polymeric film during application of the hydrophobic particles does not overly detriment the desired hydrophobic nature of the resultant polymeric film.

In order to determine how well the functionalised Al₂O₃ hydrophobic particles bonded the polymeric film was sonicated in isopropanol for approximately ten minutes and the WCA was retested.

Following sonication, the WCA of the polymeric film was 137.7° ±7.9°. It is evident that the WCA did not change significantly following sonication which indicates a strong thermal embedding of the functionalised Al₂O₃ hydrophobic particles within the polymeric film.

### Example 4

The polymeric film was heated and coated with the functionalised Fe₃O₄ particles described in Example 2 according to the method described in Example 3.

The WCA of the resultant polymeric film was 151.9° ±2.7°.

In order to determine how well the functionalised Fe₃O₄ hydrophobic particles bonded the polymeric film was sonicated in isopropanol for approximately ten minutes and the WCA was retested.

Following sonication, the WCA of the polymeric film was 90.3° ±0.5°. This represents a WCA close to that of the uncoated polymeric film. This indicates that most of the functionalised Fe₃O₄ hydrophobic particles were removed by the sonication. Without being bound to any particular theory, it is understood that functionalised Fe₃O₄ hydrophobic particles form relatively large agglomerates on the surface that are less strongly embedded than, say, functionalised Al₂O₃ hydrophobic particles. Accordingly, the functionalised Fe₃O₄ hydrophobic particles are more easily removed than the functionalised Al₂O₃ hydrophobic particles. However, that is not to say that embodiments incorporating functionalised Fe₃O₄ hydrophobic particles are not commercially viable. The sonication test merely seeks to replicate a highly destructive environment to determine the degree of bonding between the hydrophobic particles and the polymeric film. Hydrophobic films are unlikely to experience such a highly destructive environment in normal use.

### Examples 5 to 9

Bonding of the functionalised Al₂O₃ particles and the polymeric film by an epoxy resin was studied in examples 5 to 9.

In example 5, 0.08 g of epoxy resin was added to 0.66 g of the functionalised Al₂O₃ particles described in Example 1 and suspended in 40 mL of isopropanol, such that the mass ratio of functionalised Al₂O₃ particles : epoxy resin was approximately 8.6:1.0. Deposition of the mixture onto the polymeric film was performed through spray coating at ambient temperature, as described previously. Spray coating this suspension onto the polymeric film resulted in a polymeric film having a WCA of 144.3° ±4.3°.

In examples 6 to 9, the ratio of the functionalised Al₂O₃ particles and epoxy resin was adjusted.

The functionalised Al₂O₃ particle and epoxy resin ratios and the corresponding WCAs for the polymeric films of examples 5 to 9 are summarised in Table 1. Table 1 also shows the WCAs for the polymeric films after they have been sonicated in isopropanol for approximately ten minutes.

**Table 1. Water contact angle (°) before and after sonication as a function of the ratio of functionalised Al₂O₃ particles and epoxy resin.**

| Example | Functionalised Al₂O₃ particles: epoxy resin | Water contact angle (°) | Water contact angle (°) after sonication |
|---|---|---|---|
| 5 | 8.6:1.0 | 144.3 ±4.3° | 132.2 ±7.4° |
| 6 | 2.0:1.0 | 149.9 ±1.1° | 137.1 ±1.0° |
| 7 | 1.5:1.0 | 150.7 ±1.2° | 135.9 ±7.5° |
| 8 | 1.0:1.0 | 149.0 ±7.8° | 141.1 ±1.6° |
| 9 | 1.0:1.4 | 138.1 ±10.4° | 142.0 ±0.6° |

Whilst all of examples 5 to 9 achieved high WCAs, it is clear that polymeric films with the best hydrophobicity were created when the ratio of the functionalised Al₂O₃ particles and epoxy resin was from approximately 1.0:1.0 (i.e. 149.0 ±7.8°) to approximately 2.0:1.0 (i.e. 149.9 ±1.1°), e.g. 1.5:1.0 (i.e. 150.7 ±1.2°).

Moreover, as with Example 3, it is evident that the WCAs of Examples 5 to 9 did not change significantly following sonication. This appears to indicate a strong embedding of the functionalised Al₂O₃ hydrophobic particles within the epoxy resin.

### Examples 10 to 14

Bonding of the functionalised Fe₃O₄ particles and the polymeric film by an epoxy resin was studied in examples 10 to 14. The polymeric film was coated with a mixture of the epoxy resin and the functionalised Fe₃O₄ particles described in Example 2. In these examples, epoxy resin was added to the functionalised Fe₃O₄ particle suspension. Deposition of the mixture onto the polymeric film was performed through spray coating at ambient temperature, as described previously.

The functionalised Fe₃O₄ particle and epoxy resin ratios and the corresponding WCAs for the polymeric films of examples 10 to 14 are summarised in Table 2. Table 2 also shows the WCAs for the polymeric films after they have been sonicated in isopropanol for approximately ten minutes.

**Table 2. Water contact angle (°) before and after sonication as a function of the ratio of functionalised Fe₃O₄ particles and epoxy resin.**

| Example | Isostearate functionalised Fe₃O₄ particles:epoxy resin | Water contact angle (°) | Water contact angle (°) after sonication |
|---|---|---|---|
| 10 | 11.8:1.0 | 124.3 ±10.6° | 92.7 ±8.3° |
| 11 | 6.5:1.0 | 102.1 ±4.3° | 85.3 ±14.5° |
| 12 | 2.0:1.0 | 89.9 ±4.7° | 84.4 ±5.9° |
| 13 | 1.0:1.0 | 75.4 ±2.2° | 81.4 ±3.0° |
| 14 | 1.0:1.5 | 80.7 ±10.8° | 81.8 ±2.5° |

When compared with Examples 5 to 9, the WCAs of Examples 10 to 14 are not as high. However, there is a clear trend that the WCA increases when increasing the ratio of functionalised Fe₃O₄ particles to epoxy resin. Therefore, it is plausible that the WCA could exceed 140° in embodiments where the functionalised Fe₃O₄ particle to epoxy resin ratio exceeds 15.0:1.0.

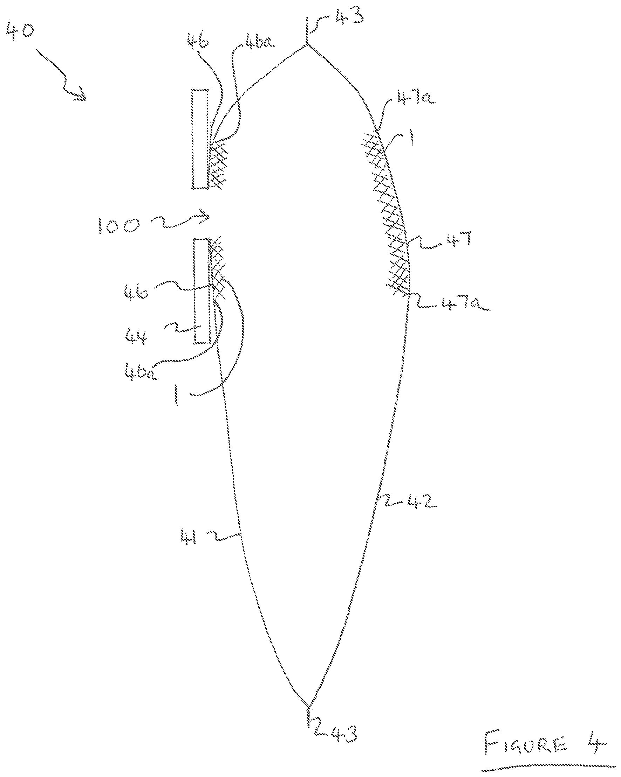

Referring now to figure 4 there is shown an ostomy pouch, indicated generally at 40, having a pair of opposed side walls 41, 42. The ostomy pouch 40 shown in this figure is of the closed-end type. The side walls 41, 42 are formed of a polymeric film, such as a polymeric film composed of any suitable heat-sealable plastic or a combination of plastics (e.g., as a coextruded laminate) that is tough, flexible, and liquid and gas impermeable. In some embodiments, the side walls 41, 42 may be separate pieces of film bonded (e.g. welded) at their respective edges 43. In other embodiments, the side walls 41, 42 may be formed from a single piece of film.

One of the side walls 41 defines a stoma-receiving opening 100 for, in use, receiving a part of a stoma (not shown) into the ostomy pouch 40. In some embodiments, the ostomy pouch 40 may comprise a wafer 44 for adhesively attaching the ostomy pouch 40 to the peristomal skin surfaces of a patient. The stoma-receiving opening 100 may have a diameter of from approximately 10 mm to approximately 50 mm to accommodate stoma having different sizes and shapes.

The ostomy pouch 40 is at least partially coated on an internal surface thereof (e.g. on an inner surface of one or both of the side walls 41, 42) with the hydrophobic particles 1. As described previously, the hydrophobic particles 1 comprise a metal oxide core 10 and a hydrocarbon chain 12 having from 6 to 32 carbon atoms. The hydrocarbon chain 12 is chemically bound to the metal oxide core 10. Any of the hydrophobic particles 1 disclosed herein could be utilised on the ostomy pouch 40.

In some embodiments, the side wall 41 defining the stoma-receiving opening 100 may comprise a first region 46 which surrounds the stoma-receiving opening 100. The first region 46 may have a peripheral edge 46a located approximately 40 mm from the centre of the stoma-receiving opening 100. Therefore, the distance across the first region 46 may be approximately 80 mm. The first region 46 may be coated with the hydrophobic particles 45.

In some embodiments, the first region 46 may only partially surround the stoma-receiving opening 100.

In some embodiments, the side wall 42 opposing the side wall 41 defining the stoma-receiving opening 100 may comprise a second region 47 which substantially faces the stoma-receiving opening 100 and is coated with the hydrophobic particles 1. The second region 47 may have a peripheral edge 47a and the distance across the second region 47 may be approximately 80 mm. Accordingly, the diameter of the second region 47 may be greater than the diameter of the stoma-receiving opening 100. The diameter of the second region 47 may also be approximately equal to the diameter of the first region 46. In some embodiments, the diameter of the second region 47 may be greater than or less than the diameter of the first region 46.

In some embodiments, the entire inner surface(s) of one or both side walls 41, 42 may constitute the respective first and second region(s) 46, 47. Accordingly, in some embodiments, the entire inner surface(s) of one or both side walls 41, 42 may be coated with the hydrophobic particles 1.

In use, body waste enters the ostomy pouch 40 from the stoma (not shown) via the stoma-receiving opening 100. As a consequence of the hydrophobic particles 1 present at the first and / or second region(s) 46, 47, and the high WCA thereof, substantial amounts of the body waste do not become attached to the side walls 41, 42 at or immediately adjacent the stoma-receiving opening 100. Instead, due to the self-cleaning nature of the hydrophobic particles 1, the body waste falls towards the bottom of the ostomy pouch 40. It has been found, particularly in relation to ostomy pouches of the closed-end type, that the phenomenon of pancaking is therefore substantially reduced. Accordingly, the adhesive of the wafer 44 does not become contaminated such that the wafer 44 detaches from the patient.

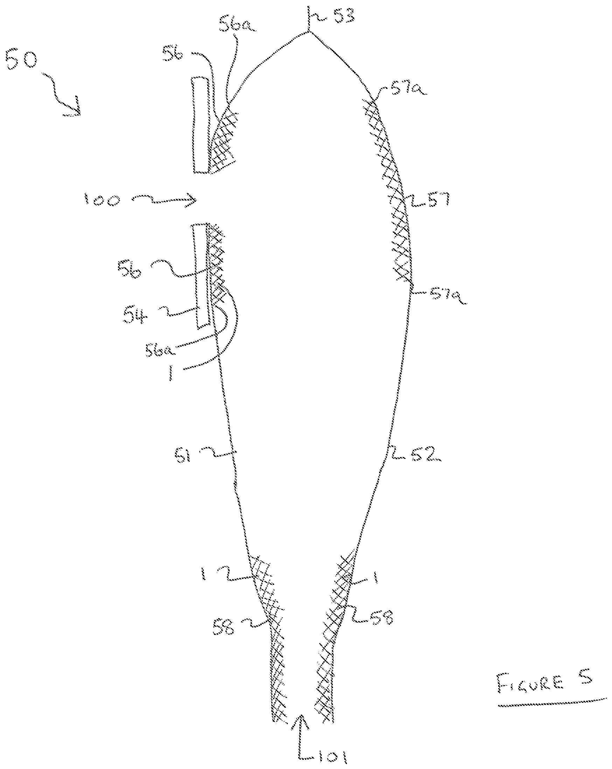

Referring now to figure 5 there is shown an ostomy pouch, indicated generally at 50. The ostomy pouch 50 shown in this figure is of the open-end type. Features of the ostomy pouch 50 that are common with the ostomy pouch 40 of figure 4 are denoted with a corresponding reference numeral in the 50s, rather than in the 40s. For instance, the side walls of the ostomy device 50 are denoted with reference numerals 51, 52, rather than 41, 42.

Embodiments of open-end type pouches may comprise a drainable opening, indicated generally at 101, at a lower end of the ostomy pouch 50 for drainage thereof. In some embodiments, the side walls 51, 52 may comprise a third region 58 which surrounds the drainable opening 101 and is coated with the hydrophobic particles 1.

In some embodiments the drainable opening 101 may be opened and closed using retaining means (not shown), such as a retaining clip or a hook and loop fastener. In such embodiments, the drainable opening 101 is typically closed by folding the side walls 51, 52 around one another and opened by reversing the action. In other embodiments the drainable opening 101 may be fitted with a valve (not shown). The internal surface of the valve may be coated with the hydrophobic particles 1.

Due to the high WCA of the hydrophobic particles 1 it has been found that the drainable opening 101 cleans itself of body waste. Accordingly, the drainable opening 101 is less likely to become clogged which can prolong the life of the ostomy pouch 50 and / or improve the hygiene thereof.

In some embodiments of the open-end type ostomy pouch 50, one or both of the first and / or second region(s) 56, 57 coated in the hydrophobic particles 1 may not be present. Accordingly, the hydrophobic particles 1 may be present only at the third region 58.

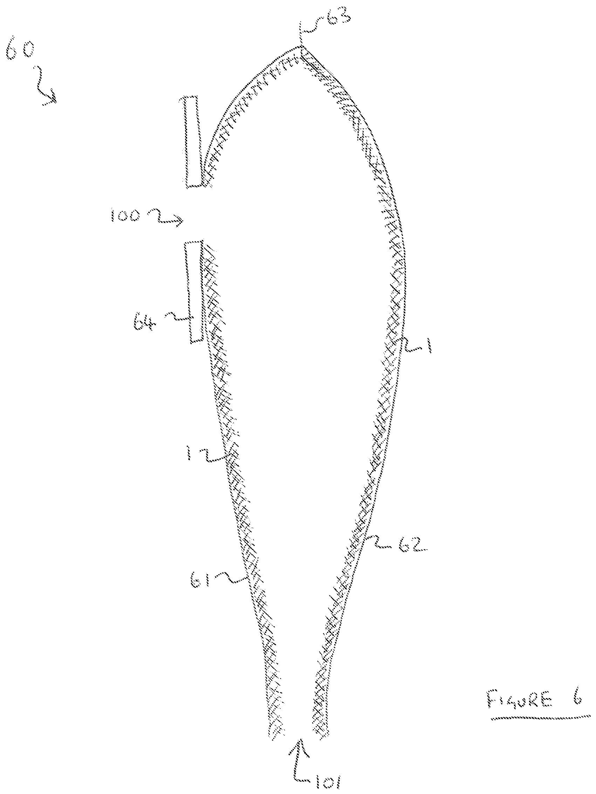

Referring now to figure 6 there is shown an ostomy pouch, indicated generally at 60. The ostomy pouch 60 shown in this figure is of the open-end type.

Features of the ostomy pouch 60 that are common with the ostomy pouch 40 of figure 4 are denoted with a corresponding reference numeral in the 60s, rather than in the 40s. For instance, the side walls of the ostomy device 60 are denoted with reference numerals 61, 62, rather than 41, 42.

The entire internal surfaces of the side walls 61, 62 are coated with the hydrophobic particles 1 in ostomy pouch 60 of figure 6. However, ostomy pouches of a closed-end type could also be configured with the entire internal surfaces coated with the hydrophobic particles, without departing from the scope of the invention.

An advantage of coating the entire internal surfaces of an ostomy pouch with the hydrophobic particles is that the ostomy pouch is generally easier to clean.

In summary, the present invention relates to ostomy pouches 40, 50, 60 formed from polymeric films that have improved self-cleaning properties by virtue of attaching hydrophobic particles 1 thereto. It has been found that the surface energies of these polymeric films are very low and that the polymeric films are non-toxic. Accordingly, not only are the ostomy pouches 40, 50, 60 improved in terms of their self-cleaning properties but they are also improved because they are non-toxic. It is also clear that the adherence of the hydrophobic particles 1 to polymeric films is improved when compared to prior art solutions. Therefore, it is understood that the ostomy pouches 40, 50, 60 of the present invention will be longer lasting, specifically in terms of their hydrophobic nature.

It may be useful to tune the WCA at different regions of the ostomy pouch 40, 50, 60. In some embodiments, the WCA of any one of the first region 46, 56, the second region 47, 57 or the third region 58 may differ from another region. One way in which this can be achieved is by varying the concentration of hydrophobic particles that are deposited at the different regions. For instance, a solution of 4 wt% of Al₂O₃ hydrophobic particles may be deposited at the first and second regions 46, 56; 47, 57 and a solution of 2 wt% Al₂O₃ hydrophobic particles may be deposited at the third region 58. Accordingly, the first and second regions 46, 56; 47, 57 will have a higher WCA than the third region 58 due to the high concentration of Al₂O₃ hydrophobic particles.

As used herein, the term "hydrocarbon chain" is intended to have its usual meaning, i.e. a molecule that consists entirely of hydrogen and carbon. Representative features are set out in the following clauses, which stand alone or may be combined, in any combination, with one or more features disclosed in the text and/or drawings of the specification.

When used in this specification and claims, the terms "comprises" and "comprising" and variations thereof mean that the specified features, steps or integers are comprised. The terms are not to be interpreted to exclude the presence of other features, steps or components.

The invention is defined in the following claims. In particular, methods relating to manufacture of ostomy pouches are not a part of the invention.

## Claims

1. An ostomy pouch (40; 50; 60) having a pair of opposed side walls (41, 42; 51, 52; 61, 62), one of the side walls defining a stoma-receiving opening (100) for, in use, receiving a part of a stoma, wherein one or both of the side walls is formed of a polymeric film (2; 20), wherein the polymeric film is at least partially coated on an internal surface thereof with hydrophobic particles (1), the hydrophobic particles comprising:
a metal oxide core (10); and
a hydrocarbon chain (12) having from 6 to 32 carbon atoms,
wherein the hydrocarbon chain is chemically bound to the metal oxide core,
**characterised in that** the hydrocarbon chain is branched.

2. An ostomy pouch (40; 50; 60) according to claim 1, wherein the side wall (41; 51; 61) defining the stoma-receiving opening (100) is formed of the polymeric film (2; 20) and comprises a first region (46; 56) which at least partially surrounds or surrounds the stoma-receiving opening (100), wherein the first region is coated with the hydrophobic particles (1).

3. An ostomy pouch (40; 50; 60) according to claim 1 or claim 2, wherein the side wall (42; 52; 62) opposing the side wall (41; 51; 61) defining the stoma-receiving opening (100) is formed of the polymeric film (2; 20) and comprises a second region (47; 57) which substantially faces the stoma-receiving opening, wherein the second region is coated with the hydrophobic particles (1); or wherein the side wall (42; 52; 62) opposing the side wall (41; 51; 61) defining the stoma-receiving opening (100) is formed of the polymeric film (2; 20) and comprises a second region (47; 57) which substantially faces the stoma-receiving opening, wherein the second region is coated with the hydrophobic particles (1) and wherein the diameter of the second region is greater than the diameter of the stoma-receiving opening.

4. An ostomy pouch (40; 50; 60) according to claim 3, when dependent on claim 2, wherein the diameter of the second region (47; 57) is approximately equal to the diameter of the first region (46; 56).

5. An ostomy pouch (50; 60) according to any preceding claim, further comprising a drainable opening (101) at a lower end thereof, both side walls (51, 52; 61, 62) being formed of the polymeric film (2; 20) and comprising a third region (58) which surrounds the drainable opening, wherein the third region is coated with the hydrophobic particles (1); or further comprising a drainable opening (101) at a lower end thereof, both side walls (51, 52; 61, 62) being formed of the polymeric film (2; 20) and comprising a third region (58) which surrounds the drainable opening (101), wherein the third region is coated with the hydrophobic particles (1) and wherein the drainable opening includes a valve, optionally wherein the internal surface of the valve is coated with the hydrophobic particles.

6. An ostomy pouch (60) according to any preceding claim, wherein both side walls (61, 62) are formed of the polymeric film (2; 20) and the entire internal surfaces thereof are coated with the hydrophobic particles (1).

7. An ostomy pouch (40; 50; 60) according to any preceding claim, wherein the average diameter of the hydrophobic particles (1) is less than or equal to approximately 200 nm; or wherein the average diameter of the hydrophobic particles (1) is less than or equal to approximately 50 nm; or wherein the average diameter of the hydrophobic particles (1) is from approximately 8 nm to approximately 20 nm.

8. An ostomy pouch (40; 50; 60) according to any preceding claim, wherein the metal oxide core (10) comprises one or a combination of aluminium oxide, iron oxide, zinc oxide, and silicon oxide.

9. An ostomy pouch (40; 50; 60) according to any preceding claim, wherein the hydrocarbon (12) chain is aliphatic.

10. An ostomy pouch (40; 50; 60) according to any preceding claim, wherein the hydrocarbon chain (12) is covalently bound to the metal oxide (10) core via a functional group (14); or wherein the hydrocarbon chain (12) is covalently bound to the metal oxide core (10) via a functional group (14) and wherein the functional group comprises any one or a combination of hydroxide, carboxylate, phosphonate, phosphinate, thiolate and thiocarboxylate.

11. An ostomy pouch (40; 50; 60) according to any preceding claim, wherein the polymeric film (2; 20) comprises a thermoplastic film; or wherein the polymeric film (2; 20) comprises a thermoplastic film and wherein the thermoplastic film comprises polyolefin, vinyl polymer or polyacetal film.

12. An ostomy pouch (40; 50; 60) according to claim 11, wherein the thermoplastic film comprises a co-extruded bilayer or multilayer film.

13. An ostomy pouch (40; 50; 60) according to any preceding claim, wherein the hydrophobic particles (1) are at least partially embedded in the polymeric film (2).

14. An ostomy pouch (40; 50; 60) according to any one of claims 1 to 12, wherein the hydrophobic particles (1) and the polymeric film (20) are secured to one another by an adhesive (30); or wherein the hydrophobic particles (1) and the polymeric film (20) are secured to one another by an adhesive (30) and wherein the mass ratio of the hydrophobic particles and the adhesive is from approximately 1.0:1.0 to approximately 2.0:1.0.

## Patentansprüche

1. Stomabeutel (40; 50; 60), der ein Paar von gegenüberliegenden Seitenwänden (41, 42; 51, 52; 61, 62) aufweist, wobei eine der Seitenwände eine Stoma-Aufnahmeöffnung (100), in Gebrauch, zur Aufnahme eines Teils eines Stomas definiert, wobei eine oder beide der Seitenwände aus einer Polymerfolie (2; 20) gebildet ist, wobei die Polymerfolie zumindest teilweise auf einer Innenfläche davon mit hydrophoben Teilchen (1) beschichtet ist, wobei die hydrophoben Teilchen umfassen:
Einen Metalloxidkern (10); und
eine Kohlenwasserstoffkette (12), die von 6 bis 32 Kohlenstoffatome aufweist,
wobei die Kohlenwasserstoffkette chemisch an den Metalloxidkern gebunden ist, **dadurch gekennzeichnet, dass** die Kohlenwasserstoffkette verzweigt ist.

2. Stomabeutel (40; 50; 60) nach Anspruch 1, wobei die Seitenwand (41; 51; 61), welche die Stoma-Aufnahmeöffnung (100) definiert, aus der Polymerfolie (2; 20) gebildet ist, und einen ersten Bereich (46; 56) umfasst, der die Stoma-Aufnahmeöffnung (100) zumindest teilweise umgibt oder umgibt, wobei der erste Bereich mit den hydrophoben Teilchen (1) beschichtet ist.

3. Stomabeutel (40; 50; 60) nach Anspruch 1 oder Anspruch 2, wobei die Seitenwand (42; 52; 62) gegenüberliegend der Seitenwand (41; 51; 61), welche die Stoma-Aufnahmeöffnung (100) definiert, aus der Polymerfolie (2; 20) gebildet ist und einen zweiten Bereich (47; 57) umfasst, welcher der Stoma-Aufnahmeöffnung wesentlich zugewandt ist, wobei der zweite Bereich mit den hydrophoben Teilchen (1) beschichtet ist; oder wobei die Seitenwand (42; 52; 62) gegenüberliegend der Seitenwand (41; 51; 61), welche die Stoma-Aufnahmeöffnung (100) definiert, aus der Polymerfolie (2; 20) gebildet ist und einen zweiten Bereich (47; 57) umfasst, welcher der Stoma-Aufnahmeöffnung wesentlich zugewandt ist, wobei der zweite Bereich mit den hydrophoben Teilchen (1) beschichtet ist, und wobei der Durchmesser des zweiten Bereichs größer als der Durchmesser der Stoma-Aufnahmeöffnung ist.

4. Stomabeutel (40; 50; 60) nach Anspruch 3, wenn von Anspruch 2 abhängig, wobei der Durchmesser des zweiten Bereichs (47; 57) annähernd gleich dem Durchmesser des ersten Bereichs (46; 56) ist.

5. Stomabeutel (50; 60) nach irgendeinem vorhergehenden Anspruch, der ferner eine dränierbare Öffnung (101) an einem unteren Ende davon umfasst, wobei beide Seitenwände (51, 52; 61, 62) aus der Polymerfolie (2; 20) gebildet sind und einen dritten Bereich (58) umfassen, der die dränierbare Öffnung umgibt, wobei der dritte Bereich mit den hydrophoben Teilchen (1) beschichtet ist; oder ferner eine dränierbare Öffnung (101) an einem unteren Ende davon umfasst, wobei beide Seitenwände (51, 52; 61, 62) aus der Polymerfolie (2; 20) gebildet sind und einen dritten Bereich (58) umfassen, der die dränierbare Öffnung (101) umgibt, wobei der dritte Bereich mit den hydrophoben Teilchen (1) beschichtet ist, und wobei die dränierbare Öffnung ein Ventil einschließt, optional wobei die Innenfläche des Ventils mit den hydrophoben Teilchen beschichtet ist.

6. Stomabeutel (60) nach irgendeinem vorhergehenden Anspruch, wobei beide Seitenwände (61, 62) aus der Polymerfolie (2; 20) gebildet sind und die ganzen Innenflächen davon mit den hydrophoben Teilchen (1) beschichtet sind.

7. Stomabeutel (40; 50; 60) nach irgendeinem vorhergehenden Anspruch, wobei der durchschnittliche Durchmesser der hydrophoben Teilchen (1) weniger als oder gleich ca. 200 nm beträgt; oder wobei der durchschnittliche Durchmesser der hydrophoben Teilchen (1) weniger als oder gleich ca. 50 nm beträgt; oder wobei der durchschnittliche Durchmesser der hydrophoben Teilchen (1) von ca. 8 nm bis ca. 20 nm beträgt.

8. Stomabeutel (40; 50; 60) nach irgendeinem vorhergehenden Anspruch, wobei der Metalloxidkern (10) einen oder eine Kombination aus Aluminiumoxid, Eisenoxid, Zinkoxid und Siliziumoxid umfasst.

9. Stomabeutel (40; 50; 60) nach irgendeinem vorhergehenden Anspruch, wobei die Kohlenwasserstoffkette (12) aliphatisch ist.

10. Stomabeutel (40; 50; 60) nach irgendeinem vorhergehenden Anspruch, wobei die Kohlenwasserstoffkette (12) über eine funktionale Gruppe (14) kovalent an den Metalloxidkern (10) gebunden ist; oder wobei die Kohlenwasserstoffkette (12) über eine funktionale Gruppe (14) kovalent an den Metalloxidkern (10) gebunden ist, und wobei die funktionale Gruppe irgendein oder eine Kombination von Hydroxid, Carboxylat, Phosphonat, Phosphinat, Thiolat und Thiocarboxylat umfasst.

11. Stomabeutel (40; 50; 60) nach irgendeinem vorhergehenden Anspruch, wobei die Polymerfolie (2; 20) eine thermoplastische Folie umfasst; oder wobei die Polymerfolie (2; 20) eine thermoplastische Folie umfasst und wobei die thermoplastische Folie Polyolefin-, Vinylpolymer- oder Polyacetalfolie umfasst.

12. Stomabeutel (40; 50; 60) nach Anspruch 11, wobei die thermoplastische Folie eine coextrudierte Doppel- oder Mehrschichtfolie umfasst.

13. Stomabeutel (40; 50; 60) nach irgendeinem vorhergehenden Anspruch, wobei die hydrophoben Teilchen (1) zumindest teilweise in die Polymerfolie (2) eingebettet sind.

14. Stomabeutel (40; 50; 60) nach irgendeinem der Ansprüche 1 bis 12, wobei die hydrophoben Teilchen (1) und die Polymerfolie (20) durch einen Klebstoff (30) einander befestigt sind; oder wobei die hydrophoben Teilchen (1) und die Polymerfolie (20) durch einen Klebstoff (30 aneinander befestigt sind und wobei das Masseverhältnis der hydrophoben Teilchen und des Klebstoffs von ca. 1,0:1,0 bis ca. 2,0:1,0 beträgt.

## Revendications

1. Poche de stomie (40 ; 50 ; 60) comportant une paire de parois latérales opposées (41, 42 ; 51, 52 ; 61, 62), l'une des parois latérales délimitant une ouverture de réception de stomie (100) pour, pendant l'utilisation, recevoir une partie d'une stomie, dans lequel une ou les deux parois latérales sont formées d'un film polymère (2 ; 20), dans lequel le film polymère est au moins partiellement revêtu sur sa surface interne de particules hydrophobes (1), les particules hydrophobes comprenant :
un noyau d'oxyde métallique (10) ; et
une chaîne hydrocarbonée (12) comportant de 6 à 32 atomes de carbone,
dans lequel la chaîne hydrocarbonée est liée chimiquement au noyau d'oxyde métallique, **caractérisée en ce que** la chaîne hydrocarbonée est ramifiée.

2. Poche de stomie (40 ; 50 ; 60) selon la revendication 1, dans lequel la paroi latérale (41 ; 51 ; 61) délimitant l'ouverture de réception de stomie (100) est formée du film polymère (2 ; 20) et comprend une première région (46 ; 56) qui entoure au moins partiellement ou entoure l'ouverture de réception de stomie (100), dans lequel la première région est revêtue des particules hydrophobes (1).

3. Poche de stomie (40 ; 50 ; 60) selon soit la revendication 1, soit la revendication 2, dans lequel la paroi latérale (42 ; 52 ; 62) opposée à la paroi latérale (41 ; 51 ; 61) délimitant l'ouverture de réception de stomie (100) est formée du film polymère (2 ; 20) et comprend une deuxième région (47 ; 57) qui est sensiblement située face à l'ouverture de réception de stomie, dans lequel la deuxième région est revêtue des particules hydrophobes (1) ; ou dans lequel la paroi latérale (42 ; 52 ; 62) opposée à la paroi latérale (41 ; 51 ; 61) délimitant l'ouverture de réception de stomie (100) est formée du film polymère (2 ; 20) et comprend une deuxième région (47 ; 57) qui est sensiblement située face à l'ouverture de réception de stomie, dans lequel la deuxième région est revêtue des particules hydrophobes (1) et dans lequel le diamètre de la deuxième région est supérieur au diamètre de l'ouverture de réception de stomie.

4. Poche de stomie (40 ; 50 ; 60) selon la revendication 3, lorsqu'elle relève de la revendication 2, dans lequel le diamètre de la deuxième région (47 ; 57) est environ égal au diamètre de la première région (46 ; 56).

5. Poche de stomie (50 ; 60) selon l'une quelconque des revendications précédentes, comprenant en outre une ouverture drainable (101) à son extrémité inférieure, les deux parois latérales (51, 52 ; 61, 62) étant formées du film polymère (2 ; 20) et comprenant une troisième région (58) qui entoure l'ouverture drainable, dans laquelle la troisième région est revêtue des particules hydrophobes (1) ; ou comprenant en outre une ouverture drainable (101) à son extrémité inférieure, les deux parois latérales (51, 52 ; 61, 62) étant formées du film polymère (2 ; 20) et comprenant une troisième région (58) qui entoure l'ouverture drainable (101), dans lequel la troisième région est revêtue des particules hydrophobes (1) et dans lequel l'ouverture drainable inclut une valve, facultativement dans lequel la surface interne de la valve est revêtue des particules hydrophobes.

6. Poche de stomie (60) selon l'une quelconque des revendications précédentes, dans lequel les deux parois latérales (61, 62) sont formées du film polymère (2 ; 20) et la totalité de leurs surfaces internes sont revêtues des particules hydrophobes (1).

7. Poche de stomie (40 ; 50 ; 60) selon l'une quelconque des revendications précédentes, dans lequel le diamètre moyen des particules hydrophobes (1) est inférieur ou égal à environ 200 nm ; ou dans lequel le diamètre moyen des particules hydrophobes (1) est inférieur ou égal à environ 50 nm ; ou dans lequel le diamètre moyen des particules hydrophobes (1) est compris entre environ 8 nm et environ 20 nm.

8. Poche de stomie (40 ; 50 ; 60) selon l'une quelconque des revendications précédentes, dans lequel le noyau d'oxyde métallique (10) comprend un oxyde ou une combinaison d'oxyde d'aluminium, d'oxyde de fer, d'oxyde de zinc et d'oxyde de silicium.

9. Poche de stomie (40 ; 50 ; 60) selon l'une quelconque des revendications précédentes, dans lequel la chaîne hydrocarbonée (12) est aliphatique.

10. Poche de stomie (40 ; 50 ; 60) selon l'une quelconque des revendications précédentes, dans lequel la chaîne hydrocarbonée (12) est liée de manière covalente au noyau d'oxyde métallique (10) par un groupe fonctionnel (14) ; ou dans lequel la chaîne hydrocarbonée (12) est liée de manière covalente au noyau d'oxyde métallique (10) par un groupe fonctionnel (14) et dans lequel le groupe fonctionnel comprend un élément ou une combinaison d'hydroxyde, carboxylate, de phosphonate, de phosphinate, de thiolate et de thiocarboxylate.

11. Poche de stomie (40 ; 50 ; 60) selon l'une quelconque des revendications précédentes, dans lequel le film polymère (2 ; 20) comprend un film thermoplastique ; ou dans lequel le film polymère (2 ; 20) comprend un film thermoplastique et dans lequel le film thermoplastique comprend un film de polyoléfine, de vinyle ou de polyacétal.

12. Poche de stomie (40 ; 50 ; 60) selon la revendication 11, dans lequel le film thermoplastique comprend un film bicouche ou multicouche coextrudé.

13. Poche de stomie (40 ; 50 ; 60) selon l'une quelconque des revendications précédentes, dans lequel les particules hydrophobes (1) sont au moins partiellement intégrées dans le film polymère (2).

14. Poche de stomie (40 ; 50 ; 60) selon l'une quelconque des revendications 1 à 12, dans lequel les particules hydrophobes (1) et le film polymère (20) sont fixée les uns aux autres par un adhésif (30) ; ou dans lequel les particules hydrophobes (1) et le film polymère (20) sont fixés les uns aux autres par un adhésif (30) et dans lequel le rapport de masse des particules hydrophobes et de l'adhésif est compris entre environ 1,0:1,0 et environ 2,0:1,0.
